# EUROPEAN PATENT SPECIFICATION

(11) **EP 2 461 870 B1**
(45) Date of publication and mention of the grant of the patent: **06.09.2017**
(21) Application number: 10802912.5
(22) Date of filing: 22.07.2010
(51) Int. Cl.: A61K 39/395, A61K 31/716, A61P 35/00, A61K 45/00

(54) **COMPOSITION COMPRISING BETA-GLUCAN AND AN EGF-R ANTIBODY OR AN EGF-R ANTAGONIST FOR USE IN THE TREATMENT OF KRAS-MUTATED TUMORS**
ZUSAMMENTSETZUNG ENTHALTEND BETA-GLUCAN UND EIN EGF-R-ANTIKÖRPER ODER EIN EGF-R INHIBITOR ZUR VERWENDUNG IN DER BEHANDLUNG VON KRAS-MUTIERTEN TUMOREN
COMPOSITION COMPRENANT BETA-GLUCANE ET UN ANTICORPS ANTI-EGFR OU UN INHIBITEUR DE L'EGFR DANS LE TRAITEMENT DES TUMEURS AVEC MUTATION KRAS

(30) Priority: 22.07.2009 US 271514 P
(43) Date of publication of application: 13.06.2012
(73) Proprietor: Biothera, Inc., Eagan, MN 55121 (US)
(72) Inventor: VASILAKOS, John, P., Woodbury MN 55125-8695 (US)
(74) Representative: Vossius & Partner Patentanwälte Rechtsanwälte mbB
(86) International application number: PCT/US2010/042925
(87) International publication number: WO 2011/011617

(56) References cited:
- WO-A2-2006/119395
- US-A1- 2005 176 015
- US-A1- 2007 071 759
- US-A1- 2009 017 024
- Tamayo et al: "A phase Ib/2, dose-escalating, safety, and efficacy study of imprime PGG, cetuximab and irinotecan in patients with advanced colorectal cancer (CRC)", Journal of Clinical Oncology, 2009 ASCO Annual Meeting Proceedings, vol. 27, no. 15S, E15062, 20 May 2009 (2009-05-20), XP002691706, Retrieved from the Internet: URL:http://meeting.ascopubs.org/cgi/conten t/abstract/27/15S/e15062 [retrieved on 2013-02-01]
- A. LIEVRE ET AL: "KRAS Mutations As an Independent Prognostic Factor in Patients With Advanced Colorectal Cancer Treated With Cetuximab", JOURNAL OF CLINICAL ONCOLOGY, vol. 26, no. 3, 20 January 2008 (2008-01-20), pages 374-379, XP055036677, ISSN: 0732-183X, DOI: 10.1200/JCO.2007.12.5906
- LI BING ET AL: "Combined yeast beta-glucan and antitumor monoclonal antibody therapy requires C5a-mediated neutrophil chemotaxis via regulation of decay-accelerating factor CD55", CANCER RESEARCH, vol. 67, no. 15, August 2007 (2007-08), pages 7421-7430, XP002691707, ISSN: 0008-5472
- LIU J ET AL: "Combined yeast-derived beta-glucan with anti-tumor monoclonal antibody for cancer immunotherapy", EXPERIMENTAL AND MOLECULAR PATHOLOGY, ACADEMIC PRESS, US, vol. 86, no. 3, 1 June 2009 (2009-06-01), pages 208-214, XP026096506, ISSN: 0014-4800, DOI: 10.1016/J.YEXMP.2009.01.006 [retrieved on 2009-01-21]

## Description

### BACKGROUND

Certain epidermal growth factor receptor (EGF-r) antagonists are established therapeutic agents effective for treating certain types of tumors. For example, cetuximab (ERBITUX, Bristol-Myers Squibb, New York, NY) and panitumumab (VECTIBIX, Amgen, Thousand Oaks, CA) are EGF-r antagonist antibodies, each of which is approved for the treatment of patients who have metastatic colorectal cancer. However, certain patients with colorectal cancer may not respond to treatment with panitumumab or cetuximab. These patients often have tumors with *KRAS* mutations.

In metastatic colorectal cancer, EGF-r transmits signals through a set of intracellular proteins, which instruct the cancer cell to reproduce and metastasize. Blocking the EGF-r-such as by using an EGF-r antagonist-can interfere with this malignant signaling. However, in patients with mutant *KRAS*, the signaling continues despite EGF-r antagonist therapy. Mutated *KRAS* genes have been reported to be detected in about 40% of metastatic colorectal cancer patients, depending on the testing used.

In addition, data from lung cancer trials to show that the *KRAS* mutation is also associated with a lack of response to the small-molecule compounds that inhibit EGF-r-e.g., erlotinib (TARCEVA, Genentech, South San Francisco, CA) and gefitinib (IRESSA, AstraZeneca, Wilmington, DE). Erlotinib is currently approved for treatment of patients with locally advanced or metastatic non-small cell lung cancer (NSCLC) or advanced, unresectable, or metastatic pancreatic cancer. Gefitinib is currently approved for the treatment of locally advanced NSCLC. The *KRAS*-related data on erlotinib and gefitinib in treating NSCLC have been reported to be consistent with the *KRAS*-related data on the treatment of colorectal cancer with cetuximab or panitumumab-for these drugs to have any benefit, the tumors must possess wild-type *KRAS*.

Consequently, a need exists for methods for treating conditions associated with *KRAS* mutations-conditions that do not respond well to EGF-r antagonist therapies.

### SUMMARY

The present invention is defined by the claims. In more detail, the present invention relates to a composition comprising a ß-glucan and a second composition that includes at least one of: an epidermal growth factor receptor (EGF-r) antagonist; and an antibody that binds to EGF-r for the use in treating a subject having a tumor comprising KRAS-mutated cells; wherein each composition is used in an amount that, in combination with the other composition, is effective to ameliorate at least one symptom or clinical sign of the tumor with the proviso that the KRAS-mutated cells are not SKOV 3 cells or NCI H23 cells. The present invention furthermore pertains to a composition comprising a ß-glucan for use in improving treatment of a tumor in a subject, wherein said subject is treated with an EGF-r antagonist, and wherein said subject comprises neoplastic cells comprising a KRAS mutation with the proviso that the condition does not comprise a tumor that comprises SKOV-3 cells or NCI-H23 cells.
Also disclosed herein are methods relating to treating a condition characterized, at least in part, by the presence of cells that include at least one KRAS mutation. Generally, the method can include administering to a subject having a tumor comprising cells comprising a KRAS mutation one or more compositions that, in total, include substances in amounts that, in combination with the other, are effective to ameliorate at least one symptom or clinical sign associated with the condition. One composition includes a β-glucan. A second composition may include an antibody composition that binds to at least one antigen specific to the KRAS-mutated cells. A second composition may also include an epidermal growth factor receptor (EGF-r) antagonist. In either case, the composition that includes a β-glucan and the second composition can be combined prior to being administered to the subject so that the subject experiences a single administration of both compositions.

The method may further include administering to the subject a composition that includes a membrane-bound complement regulatory protein (mCRP) antagonist. Prior to being administered to the subject, the composition that includes a mCRP antagonist may be combined with the composition that includes the β-glucan, the second composition, or both.

The condition can include colon carcinoma, colorectal carcinoma, lung adenocarcinoma, lung squamous cell carcinoma, lung large cell carcinoma, intraductal papillary mucinous tumor, and/or mucinous cystic tumor.

In case the second composition includes antibody, the antibody can include a monoclonal antibody, a humanized monoclonal antibody, a recombinant antibody, or any combination of two or more types of antibody. The antibody can include cetuximab.

The method can further include identifying the subject as having the condition that includes a tumor having cells that include a KRAS mutation.

Also disclosed herein is a method that generally includes providing to a subject having the condition a composition that comprises a β-glucan, wherein administering to the subject an amount of the composition that, in combination with an antibody composition that specifically binds to at least one antigen specific to the KRAS-mutated cells, is effective to ameliorate at least one symptom or clinical sign of the condition. A second composition may include an antibody composition that binds to at least one antigen specific to the KRAS-mutated cells. A second composition can also include an epidermal growth factor receptor (EGF-r) antagonist. In either case, the composition that includes a β-glucan and the second composition can be combined prior to being administered to the subject so that the subject experiences a single administration of both compositions.

Also disclosed herein is a method of improving treatment of condition that includes administering an epidermal growth factor receptor (EGF-r) antagonist to a subject. Generally, the method includes administering an effective amount of a composition that includes a β-glucan to the subject, wherein the subject comprises neoplastic cells that include a KRAS mutation.

The composition comprising the β-glucan may be combined with the EGF-r antagonist prior to being administered to the subject.

The method may further include administering to the subject a composition that includes a mCRP antagonist. The mCRP may be combined with the β-glucan, the EGF-r antagonist, or both, prior to being administered to the subject.

The condition can include colon carcinoma, colorectal carcinoma, lung adenocarcinoma, lung squamous cell carcinoma, lung large cell carcinoma, intraductal papillary mucinous tumor, and/or mucinous cystic tumor.

The EGF-r antagonist can include antibody that specifically binds EGF-r. When the EGF-r antagonist includes antibody, the antibody can include a monoclonal antibody, a humanized monoclonal antibody, a recombinant antibody, or any combination of two or more types of antibody. The antibody can include cetuximab.

### BRIEF DESCRIPTION OF THE FIGURES

Fig. 1 is a line graph showing the results of an *in vivo* xenograft model in which tumor cells harboring a *KRAS* mutation were grafted subcutaneously into mice and then subsequently treated with an EGF-r antagonist antibody, a β-glucan, a combination of the EGF-r antibody and β-glucan, or saline. Therapy was initiated after the tumor reached 350 mm³. Tumor: GEO colon carcinoma KRAS mutant; Mice: Nu/Nu; Monoclonal antibody: ERBITUX, 0.25 mg/dose; Regimen: intravenous administration, 2x/wk, 4 wks (q3d x 8 doses).
Fig. 2 shows flow cytometry data demonstrating that a β-glucan composition enhances the ability of human peripheral blood mononuclear cells (PBMCs) to kill *KRAS*-mutated tumor cells *in vitro.*
Fig. 3 shows data demonstrating that a β-glucan composition enhances the ability of human peripheral blood mononuclear cells (PBMCs) to kill *KRAS*-mutated tumor cells *in vitro.* (N=1; E:T = 25:1).
Fig. 4 shows data demonstrating that a β-glucan composition exhibits greater antitumor activity in the presence of an EGF-r antagonist antibody. (N=1; E:T = 25:1).

### DETAILED DESCRIPTION

The present invention is defined by the claims. Also described herein are methods that can address the need to provide therapies for treating conditions that do not respond well to EGF-r antagonist therapies. Such conditions can include certain cancers such as, for example, colorectal cancer, pancreatic cancer, and non-small cell lung cancer. Generally, the methods include administering to a subject a composition that includes a β-glucan and, as either a second composition or a second component of the composition, antibody that binds to at least one antigen specific to the KRAS-mutated cells and/or an EGF-r antagonist. Administering additional components and/or compositions is also possible.

The term "and/or" means one or all of the listed elements or a combination of any two or more of the listed elements.

The terms "comprises" and variations thereof do not have a limiting meaning where these terms appear in the description and claims.

Unless otherwise specified, "a," "an," "the," and "at least one" are used interchangeably and mean one or more than one.

Also herein, the recitations of numerical ranges by endpoints include all numbers subsumed within that range (e.g., 1 to 5 includes 1, 1.5, 2, 2.75, 3, 3.80, 4, 5, etc.).

For any method disclosed herein that includes discrete steps, the steps may be conducted in any feasible order. And, as appropriate, any combination of two or more steps may be conducted simultaneously.

Provided herein is a method that can ameliorate at least one symptom or clinical sign associated with a condition that is at least partially characterized by the presence of tumor cells that include a KRAS mutation. As used herein, cells that possess a KRAS mutation (or a similar phrase such as "KRAS-mutated cells") can include cells that include a mutation in the *KRAS* gene. The *KRAS* gene is one of a family (i.e., the *RAS* family) of genes that encode a family of membrane-associated GTPases, proteins that regulate signal transduction upon binding of ligand to a variety of membrane receptors. In the scientific literature, KRAS is also written as K-RAS, Ki-RAS, and V-H-Ras. Generally, the method includes administering to a subject having such a condition one or more compositions. One composition includes a β-glucan. Another composition includes antibody that binds to at least one antigen specific to the KRAS-mutated cells. Each of the β-glucan and antibody is provided in an amount that, in combination with the other, is effective to ameliorate at least one symptom or clinical sign associated with the condition.

As used herein, the term "ameliorate" can refer to any reduction in the extent, severity, frequency, and/or likelihood of a symptom or clinical sign characteristic of a particular condition. The term "symptom" can refer to any subjective evidence of disease or of a patient's condition-e.g., pain; while the terms "sign" and/or "clinical sign" can refer to an objective physical finding relating to a particular condition capable of being found by one other than the patient-e.g., tumor diameter, tumor volume, and the like.

As used herein, the term "antibody" can refer to a monoclonal antibody, a mixture of a plurality of monoclonal antibodies, one or more recombinantly-produced antibodies, one or more synthetically produced antibodies, one or more antibodies of a polyclonal composition, or any combination of two or more of the foregoing. Antibody can be specific for a particular target such as, for example, an antigen. As used herein, the term "specific" when used to characterize the affinity of antibody for a target-e.g., immunospecific-can refer to the antibody possessing a differential (e.g., preferential) or a non-general affinity for the target. For example, antibody "specific" to a particular target is antibody that binds to the target with greater affinity, to any degree, than background affinity for the target. As used herein, the term "antigen" can refer to any substance that may be bound by antibody in a manner that is immunospecific to some degree. Also, the term "specific" when used to characterize expression of an antigen by a particular cell type is an antigen that is differentially expressed by the particular cell type to any exploitable degree. Thus, an antibody composition that specifically binds to at least one antigen specific to KRAS-mutated cells refers to antibody that differentially binds to a target antigen (e.g., the antibody binds to the target antigen with an affinity that is greater than background) that is specific to KRAS-mutated cells (e.g., the antigen is expressed by KRAS-mutated cells to an exploitably greater degree than the antigen is expressed by normal cells).

The KRAS-mutated cells are cells other than SKOV-3 cells or NCI-H23 cells.

The β-glucan and compositions that include β-glucan, are described in detail below.

The antibody composition can include either a single antibody or a plurality of antibodies. The antibody composition can include a monoclonal antibody such as, for example, a humanized monoclonal antibody. The antibody can include a recombinant antibody. The antibody can include a therapeutic antibody such as, for example, cetuximab, panitumumab, rituximab, bevacizumab, trastuzumab, alemtuzumab, and the like, that have received regulatory approval for use in treating one or more conditions such as, for example, certain cancers. The antibody can include one or more polyclonal antibodies. Standard methods for producing monoclonal antibodies-including humanize monoclonal antibodies, recombinant antibodies, and polyclonal antibodies against a particular target are known in the art. The antibody composition can include any combination of two or more antibodies.

The β-glucan composition and the antibody composition may be administered separately. Also, the β-glucan composition and the second composition can be combined prior to being administered to the subject. In this way, the subject can receive the benefit of both compositions but experience only a single administration. Exemplary methods of administering the β-glucan composition and the antibody composition are described in more detail below.

The method described herein may further include administering to the subject a composition that includes a membrane-bound complement regulatory protein (mCRP) antagonist. A "mCRP antagonist" can refer to a compound that can combine with a mCRP to inhibit biological and/or biochemical activity of the mCRP. A mCRP antagonist may be a ligand that directly binds to the mCRP. "Inhibit" and variations thereof can refer to any measurable reduction of biological and/or biochemical activity. For example, inhibition of a mCRP can refer to a decrease in the extent to which mCRP inhibits complement activation-i.e., mCRP inhibition results in increased complement activation. The extent of inhibition may be characterized as a percentage of a normal level of activity.

The mCRP antagonist can be provided in a composition separate from the β-glucan composition and/or the antibody composition. Alternatively, the mCRP antagonist, when present, can be combined with the β-glucan composition, the antibody composition, or both.

The condition can include certain forms of cancer such as, for example, colon carcinoma, colorectal carcinoma, lung cancer (e.g., lung adenocarcinoma, lung squamous cell carcinoma and/or lung large cell carcinoma), breast cancer (e.g., intraductal papillary mucinous tumor and/or mucinous cystic tumor), oral cancer, ovarian cancer, prostate cancer, pancreatic cancer, multiple myeloma, malignant melanoma and/or non-melanoma skin cancers.

The KRAS-mutated cells may include GEO cells and/or HCT-115 cells.

The antibody may bind to a KRAS-specific antigen such as, for example, EGF-r. Antibodies that bind to EGF-r, including therapeutic antibodies that have received regulatory approval for the treatment of certain conditions, are described in more detail below.

Despite the effectiveness of antibodies that bind to EGF-r for treating some cancer patients, certain patients may not respond to treatment with, for example, panitumumab or cetuximab. These patients often have tumors that harbor *KRAS* mutations. Thus, the method can include improving the efficacy of anti-EGF-r antibody therapy-i.e., improving treatment of condition that includes administering an anti-EGF-r antibody to a patient where the anti-EGF-r antibody, alone, in insufficiently effective. Generally, the method includes administering an effective amount of a composition that includes a β-glucan to a patient that has a condition characterized, at least in part, by neoplastic cells that harbor a KRAS mutation and for whom anti-EGF-r antibody therapy has been unsuccessful.

Thus, the method can further include identifying a patient as having a condition that is characterized, at least in part, by the presence of cells that harbor a KRAS mutation. Standard methods for detecting a KRAS mutation in a cell are known in the art. For example, KRAS mutations may be identified from cancer tissue or cells obtained from patients using a DNA-based assay that detects mutations in the *KRAS* gene. Such assays may be PCR-based such as, for example, the TheraScreen: KRAS Mutation Test Kit from DxS Ltd, Manchester, UK. The TheraScreen: KRAS Mutation Test Kit is approved by the FDA for testing clinical samples for KRAS mutations.

Also provided herein is a method that can ameliorate at least one symptom or clinical sign associated with a condition that is at least partially characterized by the presence of tumor cells that include a KRAS mutation. Generally, the method includes administering to a subject having such a condition one or more compositions. One composition includes a β-glucan. Another composition includes an EGF-r antagonist. Each of the β-glucan and the EGF-r antagonist is provided in an amount that, in combination with the other, is effective to ameliorate at least one symptom or clinical sign associated with the condition.

As used herein, an "EGF-r antagonist" can include a compound that can combine with EGF-r to inhibit cellular activity (e.g., cell signaling) that would otherwise result from an agonist-EGFr interaction. The extent of inhibition may be characterized as a percentage of a normal level of activity.

EGF-r antagonists include antibodies that bind to EGF-r (e.g., cetuximab and panitumumab) as well as small molecules antagonists such as, for example, erlotinib and gefitinib.

The KRAS-mutated cells are cells other than SKOV-3 cells or NCI-H23 cells.

The β-glucan, compositions that include β-glucan, and exemplary EGF-r antagonists are described in detail below. The EGF-r antagonist may include antibody that binds to EGF-r. An EGF-r antagonist antibody can include either a single antibody or a plurality of antibodies. An EGF-r antagonist antibody can include a monoclonal antibody such as, for example, a humanized monoclonal antibody. The EGF-r antagonist antibody can include a recombinant antibody. The EGF-r antagonist antibody can include a therapeutic antibody such as, for example, cetuximab, panitumumab, rituximab, bevacizumab, trastuzumab, alemtuzumab, and the like, that have received regulatory approval for use in treating one or more conditions such as, for example, certain cancers. The EGF-r antagonist antibody can include one or more polyclonal antibodies. Standard methods for producing monoclonal antibodies-including humanize monoclonal antibodies, recombinant antibodies, and polyclonal antibodies against a particular target are known in the art. The EGF-r antagonist composition can include any combination of two or more antibodies.

The β-glucan composition and the EGF-r antagonist composition may be administered separately. Also, the β-glucan composition and the EGF-r antagonist composition can be combined prior to being administered to the subject. In this way, the subject can receive the benefit of both compositions but experience only a single administration. Exemplary methods of administering the β-glucan composition and the EGF-r composition are described in more detail below.

The method may further include administering to the subject a composition that includes a mCRP antagonist. The mCRP antagonist can be provided in a composition separate from the β-glucan and/or the EGF-r antagonist. Alternatively, the mCRP antagonist, when present, can be combined with the β-glucan composition, the EGF-r antagonist composition, or both.

The condition can include certain forms of cancer such as, for example, colon carcinoma, colorectal carcinoma, lung cancer (e.g., lung adenocarcinoma, lung squamous cell carcinoma and/or lung large cell carcinoma), breast cancer (e.g., intraductal papillary mucinous tumor and/or mucinous cystic tumor), oral cancer, ovarian cancer, prostate cancer, pancreatic cancer, multiple myeloma, malignant melanoma and/or non-melanoma skin cancers. The KRAS-mutated cells may include GEO cells and/or HCT-115 cells.

Despite the effectiveness of EGF-r antagonists for treating some cancer patients, certain patients may not respond to treatment with, for example, panitumumab or cetuximab. These patients often have tumors that harbor *KRAS* mutations. Thus, the method can include improving the efficacy of EGF-r antagonist therapy-i.e., improving treatment of condition that includes administering an EGF-r antagonist to a patient where the EGF-r antagonist, alone, in insufficiently effective. Generally, the method includes administering an effective amount of a composition that includes a β-glucan to a patient that has a condition characterized, at least in part, by neoplastic cells that harbor a KRAS mutation and for whom EGF-r antagonist therapy has been unsuccessful.

Also provided herein is a method that includes, generally, providing a composition that includes a β-glucan to a subject having a condition that is characterized, at least in part, by a tumor that includes KRAS-mutated cells, wherein administering to the subject an amount of the composition that, in combination with a second composition, is effective to ameliorate at least one symptom or clinical sign of the condition, wherein the second composition includes an EGF-r antagonist and/or antibody that binds to at least one antigen specific to the KRAS-mutated cells.

The β-glucan composition may further include a mCRP antagonist.

The second composition can include any one or more of the EGF-r antagonists described herein, any one or more of the antibody compositions that include antibody that binds to at least one antigen specific to the KRAS-mutated cells described herein, or any combination thereof. The second composition can further include a mCRP antagonist.

The β-glucan composition and the second composition may be provided separately. Also, the β-glucan composition and the second composition can be combined prior to being provided to the subject.

The second composition can include antibody that binds to EGF-r such as, for example, a therapeutic antibody such as, for example, cetuximab or panitumumab that have received regulatory approval for use in treating one or more conditions such as, for example, certain cancers.

The second composition can include antibody that binds to at least one antigen specific to KRAS-mutated cells such as, for example, cetuximab, panitumumab, rituximab, bevacizumab, trastuzumab, alemtuzumab, and the like.

The KRAS-mutated cells may be cells other than SKOV-3 cells or NCI-H23 cells.

The various compositions described herein may, if desired, be provided in a pack or dispenser device and/or a kit which may contain one or more unit dosage forms containing the active ingredients. The pack may, for example, include metal or plastic foil such as, for example, a blister pack. The pack or dispenser device may be accompanied by instructions for administration.

A composition that includes β-glucan can include PGG (poly-(1-6)-β-D-glucopyranosyl-(1-3)-β-D-glucopyranose), neutral soluble β-glucan, triple helical β-glucan (BETAFECTIN, Biopolymer Engineering, Inc., Eagan, MN), β-glucans of various aggregate numbers, and any combination thereof.

The β-glucan preparations may be prepared from insoluble glucan particles. A β-glucan polysaccharide can exist in one of at least four distinct conformations: single disordered chains, single helix, single triple helix, and triple helix aggregates. As used herein, the term "single triple helix" refers to a β-glucan conformation in which three single chains are joined together to form a triple helix structure. In a single triple helix conformation, there is no higher ordering of the triple helices-i.e., there is substantially no aggregation of triple helices. As used herein, the term "triple helix aggregate" refers to a β-glucan conformation in which two or more triple helices are joined together via non-covalent interactions. A β-glucan composition can include one or more of these forms, depending upon such conditions as pH and temperature.

As used herein, the "molecular weight" of a β-glucan composition is the mass average molar mass of the collection of polymer molecules within the composition. The characterization of a collection of polymer molecules in terms of polymer mass average molar mass is well known in the art of polymer science. The terms "neutral soluble β-glucan" and "neutral soluble glucan" refer to an aqueous soluble β-glucan having a unique triple helical conformation that results from the denaturation and re-annealing of aqueous soluble glucan.

The β-glucan may be formed from starting material that includes glucan particles such as, for example, whole glucan particles described by U.S. Patent No. 4,810,646; U.S. Patent No. 4,992,540; U.S. Patent No. 5,082,936; and/or U.S. Patent No. 5,028,703. The source of the whole glucan particles can be the broad spectrum of glucan-containing fungal organisms that contain β-glucans in their cell walls. Suitable sources for the whole glucan particles include, for example, *Saccharomyces cerevisiae* R4 (deposit made in connection with U.S. Pat. No. 4,810,646; Agricultural Research Service No. NRRL Y-15903) and R4 Ad (American Type Culture Collection, Manassas, VA, ATCC No. 74181). The structurally modified glucans hereinafter referred to as "modified glucans" derived from *S. cerevisiae* R4 can be potent immune system activators (U.S. Patent No. 5,504,079).

The whole glucan particles from which the β-glucan may be prepared can be in the form of a dried powder, as described in U.S. Patent No. 4,810,646; U.S. Patent No. 4,992,540; U.S. Patent No. 5,082,936; and/or U.S. Patent No. 5,028,703. In order to prepare the β-glucan, however, it is not necessary to perform the final organic extraction and wash steps described in one or more of these patent documents.

Soluble glucans can include branched polymers of glucose, referred to as PGG, containing β(1-3) and β(1-6) linkages in varying ratios depending on the organism from which they are obtained and the processing conditions employed. PGG glucan preparations can contain neutral glucans, which have not been modified by substitution with functional (e.g., charged) groups or other covalent attachments. The biological activity of PGG glucan can be controlled by varying the average molecular weight and the ratio of β(1-6) to β(1-3) linkages of the glucan molecules. (U.S. Patent No. 4,810,646; U.S. Patent No. 4,992,540; U.S. Patent No. 5,082,936; and U.S. Patent No. 5,028,703). The average molecular weight of soluble glucans generally can be from about 10,000 daltons to about 500,000 daltons. The average molecular weight of soluble glucans can be from about 30,000 daltons to about 50,000 daltons.

Soluble β-glucan (e.g., IMP RIME PGG, Biopolymer Engineering, Inc., Eagan, MN) has been shown to increase the number of neutrophils and monocytes as well as their direct antitumor activity (e.g., phagocytosis and microbial killing). However, the soluble β-glucan does not stimulate the production of biochemical mediators, such as, for example, IL-1, TNF, and leukotrienes. When produced, these biological mediators can cause side effects such as high fever, inflammation, wasting disease, and organ failure. Also, soluble β-glucan lacks the toxicity common to many immunomodulators.

Soluble β-glucans can be composed of glucose monomers organized as a β(1-3) linked glucopyranose backbone with periodic branching via β(1-6) glycosidic linkages. The soluble glucan preparations contain glucans that have not been substantially substituted with functional (e.g., charged) groups or other covalent attachments. One biologically active form of soluble β-glucan is produced by dissociating the native glucan conformations and then re-annealing and purifying the resulting triple helical conformation. The triple helical conformation of the soluble glucan contributes to the glucan's ability to selectively activate the immune system without stimulating the production of detrimental biochemical mediators. Methods of making soluble β-glucans are described in Example 1.

Soluble glucan preparations can be prepared from insoluble glucan particles such as, for example, insoluble glucan particles derived from yeast organisms as described herein. Other strains of yeast from which insoluble glucan particles can be obtained include, for example, *Saccharomyces delbrueckii, Saccharomyces rosei, Saccharomyces microellipsodes, Saccharomyces carlsbergensis, Schizosaccharomyces pombe, Kluyveromyces lactis, Kluyveromyces fragilis, Kluyveromyces polysporus, Candida albicans, Candida cloacae, Candida tropicalis, Candida utilis, Hansenula wingeri, Hansenula arni, Hansenula henricii, Hansenula americana.*

As described above, certain conformation of β-glucan can include aggregates of single chain such as, for example, a single triple helix (an aggregate of three single helices) or a triple helix aggregate (an aggregate of triple helices). The "aggregate number" of a β-glucan conformation is the number of single chains which are joined together in that conformation. For example, the aggregate number of a single helix is 1, the aggregate number of a single triple helix is 3, and the aggregate number of a triple helix aggregate is greater than 3. For example, a triple helix aggregate consisting of two triple helices joined together has an aggregate number of 6.

The aggregate number of a β-glucan sample under a specified set of conditions can be determined by determining the average molecular weight of the polymer under those conditions. The β-glucan is then denatured, that is, subjected to conditions which separate any aggregates into their component single polymer chains. The average molecular weight of the denatured polymer is then determined. The ratio of the molecular weights of the aggregated and denatured forms of the polymer is the aggregate number. A typical β-glucan composition includes molecules having a range of chain lengths, conformations and molecular weights. Thus, the measured aggregate number of a β-glucan composition is the mass average aggregate number across the entire range of β-glucan molecules within the composition. It is to be understood that any reference herein to the aggregate number of a β-glucan composition refers to the mass average aggregate number of the composition under the specified conditions. The aggregate number of a composition indicates which conformation is predominant within the composition. For example, a measured aggregate number of about 6 or more is characteristic of a composition in which the β-glucan is substantially in the triple helix aggregate conformation. Methods for preparing β-glucan aggregates are described in Example 2.

The conformation of a PGG-glucan preparation can be temperature dependent; PGG-glucan can be predominantly in a triple helix aggregate conformation at 25°C, but can be a mixture of triple helix aggregates and the single triple helix conformation 37°C. (U.S. Patent Application Publication No. 2007/0059310 A1).

The soluble β-glucan can be substantially in a triple helix aggregate conformation under physiological conditions-e.g., physiological pH, about pH 7, and physiological temperature, about 37°C. In one example, the β-glucan consists essentially of β-glucan chains in one or more triple helix aggregate conformations under physiological conditions.

As used herein, a soluble β-glucan composition is "substantially in a triple helix conformation" if greater that about 50% by weight of the composition is in a triple helix aggregate conformation under physiological conditions. The amount of β-glucan in a triple helix aggregate conformation under physiological conditions can be greater than about 60% by weight such as, for example, greater than about 70% by weight. The soluble β-glucan composition can be characterized by an aggregate number under physiological conditions of greater than about 6 such as, for example, an aggregate number of the β-glucan composition under physiological conditions of at least about 7, at least about 8, or at least about 9.

The methods described herein enhance the immune response in humans and animals. Treatment of a condition characterized, at least in part, by the presence of a tumor that includes KRAS-mutated cells can involve activating the patient's immune system against the KRAS-mutated cells. As used herein, "treat," "treating," and variations thereof can refer to reducing, limiting progression, ameliorating, and/or resolving, to any extent, the symptoms or clinical signs related to a condition. As used herein, "treatment" can refer to any method for treating a condition. In certain contexts, "treatment" may refer to any single step of such a method such as, for example, administering a dose of a composition.

The β-glucan can be formulated for administering the β-glucan to a subject. The formulation can include one or more suitable carriers or excipients.

The β-glucan composition generally can be administered to an animal or a human in an amount that, in combination with the second composition, is effective to ameliorate at least one symptom or clinical sign of the condition. The mode of administration of the β-glucan can be oral, enteral, parenteral, intravenous, subcutaneous, intraperitoneal, intramuscular, topical, or intranasal. The form in which the β-glucan can be administered (e.g., powder, tablet, capsule, solution, emulsion) can be influenced by the route by which it is administered. The quantity of β-glucan to be administered can be determined on an individual basis, and can be at least in part influenced by the severity of the condition, the patient's condition and/or overall health, the patient's weight, and the identity and efficacy of other components of the treatment (e.g., EGF-r antagonist composition, antibody composition, mCRP antagonist, etc.). In general, a single dose can contain approximately 0.01 mg to approximately 10 mg of modified glucan per kilogram of body weight (mg/kg), although the methods described herein can be practiced using doses of β-glucan outside of this range. A single dose of β-glucan can include from about 0.1 mg/kg to about 2.5 mg/kg such as, for example, from about 0.25 mg/kg to about 2 mg/kg.

The dosage for topical application can be from about 0.001 mg to about 2 mg per milliliter of carrier (mg/ml), although the methods described herein can be practiced using a concentration of β-glucan outside of this range. The methods may include a topical dose of, for example, from about 0.01 mg/ml to about 0.5 mg/ml.

The β-glucan composition generally can be administered parenterally by injection such as, for example, subcutaneously, intravenously, intramuscularly, intraperitoneally, topically, orally or intranasaly. The β-glucan composition can be administered as a solution having a β-glucan concentration of from about 1 mg/ml to about 100 mg/ml such as, for example, a β-glucan concentration of 1 mg/ml to about 20 mg/ml or, for example, a β-glucan concentration of 1 mg/ml to about 5 mg/ml. The solvent can be a physiologically acceptable aqueous medium such as, for example, water, saline, PBS, or a 5% dextrose solution.

In general, the composition described herein can be administered to an individual periodically as necessary to ameliorate at least one symptom or clinical sign of the condition. An individual skilled in the medical arts will be able to determine the length of time during which the composition-or, in cases in which more than one composition is administered, each composition-is administered and the dosage, depending on the physical condition of the patient and the disease or disorder being treated.

The β-glucan composition can optionally include other components. The other components that can be included in a particular composition can be influenced by the manner in which the composition is to be administered. For example, a composition to be administered orally in tablet form can include, in addition to neutral soluble β-glucan, a filler (e.g., lactose), a binder (e.g., carboxymethyl cellulose, gum arabic, gelatin), an adjuvant, a flavoring agent, a coloring agent and a coating material (e.g., wax or plasticizer). A β-glucan composition to be administered in liquid form can include neutral soluble β-glucan and, optionally, an emulsifying agent, a flavoring agent, and/or a coloring agent. A β-glucan composition for parenteral administration can be mixed, dissolved, or emulsified in water, sterile saline, phosphate buffered saline (PBS), dextrose or other biologically acceptable carrier. A composition for topical administration can be formulated into a gel, ointment, lotion, cream, or other form in which the composition is capable of coating the site to be treated.

The second composition, whether an EGF-r antagonist or antibody that binds to an antigen that is specific to KRAS-mutated cells, can be formulated for administering the second composition to a subject. The formulation can include one or more suitable carriers or excipients such as, for example, those just described in connection with the β-glucan composition.

The second composition can include antibody that binds to an antigen specific to KRAS-mutated cells. As indicated above, the term "specific" when used to characterize expression of an antigen by a particular cell type is an antigen that is differentially expressed by the particular cell type to any exploitable degree. One such antigen is EGF-r, which is expressed to a greater degree in tumor cells-including KRAS-mutated tumor cells-than normal (i.e., nontumor) cells.

The second composition can include one or more EGF-r antagonists. Suitable EGF-r antagonists used in the composition described herein include polyclonal and monoclonal antibodies, recombinant human/mouse chimeric monoclonal antibody (e.g., cetuximab), antibody fragments, other proteins, and small molecules that bind specifically to the extracellular domain of the human epidermal growth factor receptor. The EGF-r antagonists can be administered separately or in various combinations.

Suitable EGF-r antagonists include anti-EGF-r antibodies such as, for example, cetuximab (ERBITUX, Bristol-Myers Squibb, New York, NY) and panitumumab (VECTIBIX, Amgen, Thousand Oaks, CA). Cetuximab is a recombinant, mouse/human chimeric monoclonal antibody which binds to the extracellular domain of the human EGF-r, blocking the binding of EGF to its receptor, thereby inhibiting growth in cells which express EGF-r, such as tumor cells. U.S. Patent No. 6,217,866 discloses two monoclonal antibodies numbered 96 and 108 from cell lines ATCC HB 9763 and 9764, respectively. Both antibodies are contemplated as EGF-r antagonists suitable for use in the methods described herein. Panitumumab is a fully human monoclonal antibody against human EGF-r described in U.S. Patent No. 6,235,883.

Suitable EGF-r antagonists also include other monoclonal antibodies, polyclonal antibodies, antibody fragments, or other proteins or small molecules. Suitable EGF-r antagonists include compounds that can combine with the EGF-r to inhibit cellular activity (e.g., cell signaling) that would otherwise result from an agonist-receptor interaction.

The EGF-r antagonist can be monoclonal or polyclonal antibody that bind to the EGF-r of various species including, for example, rat, mouse, horse, cow, goat, sheep, pig and/or rabbit. The EGF-r antagonist can be a chimeric antibody (e.g., cetuximab) produced from a human antibody and a monoclonal antibody made in any of the animals identified above. An EGF-r antagonist can include an antibody fragment such as, for example, a variable region from an antibody that binds to EGF-r. An EGF-r antagonist can include an epidermal growth factor (EGF) mutant that, while still able to bind to EGF-r, the binding of the mutant EGF to EGF-r does not result in EGF-r-mediated cell signaling and blocks binding of wild-type EGF to EGF-r. The EGF-r antagonist can be a small molecule that binds to EGF-r and blocks EGF signaling through the EGF-r such as, for example, erlotinib (TARCEVA, Genentech, South San Francisco, CA) and gefitinib (IRESSA, AstraZeneca, Wilmington, DE). The EGF-r antagonist can include a soluble EGF-r fragment such as, for example, a fragment encompassing the extracellular domain of the EGF-r, which can bind EGF thereby limiting the amount of free EGF available to bind to EGF-r.

Certain EGF-r antagonists have been granted regulatory approval for treating cancer. For example, cetuximab has received regulatory approval for treating colon cancer. Other cancers that can be treated by EGF-r antagonists include, for example, ovarian, breast, prostate, colon, pancreatic, oral epidermoid, multiple myeloma, malignant melanoma, and non-melanoma skin cancers. As described in the Background section, EGF-r antagonists, alone, can be ineffective for treating a condition characterized, at least in part, by a tumor that includes KRAS-mutated cells. Indications treatable by practicing the methods described herein include forms of the identified cancers that are characterized, at least in part, by tumors that include cells harboring at least one KRAS mutation.

The EGF-r antagonist may be administered as directed for the particular EGF-r antagonist. For example, the recommended dose of cetuximab is 400 mg/m² as an initial loading dose (first infusion) administered as a 120-minute intravenous (IV) infusion (maximum infusion rate 5 ml/min). The recommended weekly maintenance dose (all other infusions) is 250 mg/m² infused over 60 minutes (maximum infusion rate 5 ml/min). Following a two-hour infusion of 400 mg/m² of cetuximab, the maximum mean serum concentration (Cmax) was 184 µg/ml (range: 92-327 µg/ml) and the mean elimination half-life was 97 hours (range 41-213 hours). A one-hour infusion of 250 mg/m² produced a mean Cmax of 140 µg/mL (range 120-170 µg/ml). Following the recommended dose regimen (400 mg/m² initial dose/250 mg/m² weekly dose), cetuximab concentrations reached steady-state levels by the third weekly infusion with mean peak and trough concentrations across studies ranging from 168 to 235 and 41 to 85 µg/ml, respectively. The mean half-life was 114 hours (range 75-188 hours). Other administration methods are contemplated as described below.

The methods described herein involve administering to a subject a combination of a β-glucan composition and a second composition that includes either an EGF-r antagonist or antibody that binds to an antigen specific to KRAS-mutated cells. Any of the above described β-glucans can be combined with any of the above described EGF-r antagonists or any antibody that binds to any antigen specific to KRAS-mutated cells. As discussed above, the compositions can be combined prior to being administered to a subject. The combination of the β-glucan and the second composition can ameliorate at least one symptom or clinical sign of the condition, particularly when an EGF-r antagonist, if administered alone or in combination with some other active agent, is insufficient to do so. A subject may be first identified (e.g., diagnosed) as having a condition that is characterized, at least in part, by a tumor having cells that harbor at least one KRAS mutation.

The methods described herein can include administering to the subject one or more additional components. As discussed above, the additional component may be provided in a separate composition or may be combined with the β-glucan composition, the second composition, or both.

Suitable additional components can include anti-cancer drugs such as, for example, doxorubicin, cisplatin, and/or irinotecan. Other suitable anti-cancer drugs include, for example, taxanes, nitrogen mustards, ethylenimine derivatives, alkyl sulfonates, nitrosoureas, triazenes, folic acid analogs, pyrimidine analogs, purine analogs, vinca alkaloids, antibiotics, enzymes, platinum coordination complexes, substituted urea, methyl hydrazine derivatives, adrenocortical suppressants, or antagonists. More specifically, the chemotherapeutic agents can include at least one steroid, at least one progestin, at least one estrogen, at least one antiestrogen, at least one androgen, or any combination thereof. Even more specifically, the chemotherapy agents can include azaribine, bleomycin, bryostatin-1, busulfan, carmustine, chlorambucil, CPT-11, cyclophosphamide, cytarabine, dacarbazine, dactinomycin, daunorubicin, dexamethasone, diethylstilbestrol, doxorubicin, ethinyl estradiol, etoposide, fluorouracil, fluoxymesterone, gemcitabine, hydroxyprogesterone caproate, hydroxyurea, L-asparaginase, leucovorin, lomustine, mechlorethamine, medroprogesterone acetate, megestrol acetate, melphalan, mercaptopurine, methotrexate, methotrexate, mithramycin, mitomycin, mitotane, phenyl butyrate, prednisone, procarbazine, semustine streptozocin, tamoxifen, taxanes, taxol, testosterone propionate, thalidomide, thioguanine, thiotepa, uracil mustard, vinblastine, vincristine, or any combination of thereof.

Suitable additional components include, for example mCRP antagonists. Membrane-bound complement regulatory proteins (mCRPs) can limit complement-dependent cytotoxicity. Some tumors are known to overexpress one or more mCRPs, thereby interfering with complement-dependent cytotoxicity directed against the tumor. Membrane-bound complement regulatory proteins include, for example, CD46, CD55 and CD59.

CD46 (membrane cofactor protein, MCP) inhibits C3b activity. It serves as a receptor for seven human pathogens but was initially discovered as a widely expressed C3b- and C4b-binding protein. It was later shown to be a cofactor for the serine protease factor I to inactivate by limited proteolysis C3b- and C4b-binding proteins and components of the convertases. CD46 is involved in protecting normal cells from excessive complement activation. A heterozygous deficiency of CD46 can predispose an individual to hemolytic uremic syndrome.

CD55 (decay accelerating factor, DAF) inhibits C3 convertases. It was first recognized as a species restricting factor operating at the level of C3 activation. It binds C3b and C4b to inhibit the formation and reduce the half-life of the C3 convertases. CD55 is broadly distributed among cells in contact with serum, including both hematopoietic and non-hematopoietic cells. Expression of CD55 on NK cells or their targets has been associated with reduced efficiency of cell lysis. CD55 is involved in regulating the deposition of C3 on nucleated cells.

CD59 (1F-5Ag, H19, HRF20, MACIF, MIRL, P-18, Protectin) can inhibit the formation of the membrane attack complex (MAC). CD59 can block the formation of MAC by preventing development of a hydrophobic region on the C9 molecule, important for insertion into the target cell membrane. A single C9 molecule may bind to the C5b-8 complex, but CD59 can interfere with unfolding of the C9 molecule that is necessary to permit binding of multiple subsequent C9 molecules and MAC assembly. In addition, CD59 can prevent ion channel formation by the C5b-8 complex and the small-size MAC C5b-C9 complex, which in turn prevents leaky pore formation.

The methods described herein can be effective for ameliorating at least one symptom or clinical sign associated with cancer such as, for example, blood in the urine; pain and/or burning upon urination; frequent urination; cloudy urine; other problems with urination; pain in the bone and/or swelling around the affected site; bone fractures; weakness; fatigue; repeated infections; nausea; vomiting; constipation; bumps and/or bruises that persist; dizziness; drowsiness; abnormal eye movements and/or changes in vision; weakness and/or loss of feeling in the arms and/or legs; difficulties walking; fits and/or convulsions; changes in personality, memory and/or speech; headaches that tend to be worse in the morning and ease during the day, accompanied by nausea and/or vomiting; a lump or thickening of the breast; discharge from the nipple; change in the skin of the breast; a feeling of heat and/or enlarged lymph nodes under the arm; rectal bleeding (red blood in stools or black stools); abdominal cramps; constipation alternating with diarrhea; unexplained weight loss; loss of appetite; pallid complexion; dull ache and/or pain in the back and/or side; a lump in kidney area, sometimes accompanied by high blood pressure and/or abnormality in red blood cell count; fever and/or flu-like symptoms; bruising and/or prolonged bleeding; enlarged lymph nodes, spleen, and/or liver; pain in bones and/or joints; frequent infections; night sweats; wheezing; persistent cough for months; blood-streaked sputum; persistent ache in chest; congestion in lungs; change in mole and/or other bump on the skin, including bleeding or change in size, shape, color, or texture; painless swelling in the lymph nodes in the neck, underarm, or groin; persistent fever; itchy skin and/or rash; small lumps in skin; abdominal swelling; a lump in the mouth; ulceration of the lip, tongue and/or inside of the mouth that does not heal within a couple of weeks; dentures that no longer fit well; oral pain; foul breath; loose teeth; changes in speech; abnormal vaginal bleeding; digestive discomfort; intolerance of fatty foods; yellowing of the skin; abdominal masses; tenderness over the bladder; indigestion or heartburn; bloating after meals; a watery bloody discharge in postmenopausal women; pain during sexual intercourse; and/or pelvic pain.

Accordingly, the methods described herein can allow the β-glucan and the second composition to be administered in a combination that improves efficacy of the active agent (i.e., EGF-r antagonist or antibody that binds to an antigen specific to KRAS-mutated cells) in the second composition and may reduce the likelihood and/or extent of undesirable side effects of the active agent in the second composition. Side effects associated with administering an EGF-r antagonist include, for example, airway obstruction, including bronchospasm, stridor, or hoarseness; urticaria; hypotension; interstitial lung disease; inflammation; renal pathology; acneform rash; dry skin; skin fissures, fever; sepsis; kidney failure; pulmonary embolus; dehydration; diarrhea; abdominal pain; vomiting; and/or inflammatory and/or infectious sequelae such as, for example plepharitis, cheilitis, cellulites and/or cyst. Side-effects associated with the administration of EGF-r antagonist may be reduced in severity and/or frequency by coadministering the EGF-r antagonist with β-glucan.

When the methods described herein involve administering a β-glucan composition and a second composition to a subject, the administration of the compositions can include sequential or simultaneous administration. In case the compositions were to be combined prior to being administered to the subject, simultaneous administration is self-evident. However, simultaneous-or substantially simultaneous-administration of the compositions also can occur when the β-glucan composition and the second composition are separate. Substantially simultaneous administration can be accomplished, for example, by administering to the subject a single infusion that includes both a β-glucan and the second composition or in multiple, substantially simultaneous injections in which each injection includes administering a single composition. When administered separately, each composition can independently be administered by the same route or by different routes. For example, the β-glucan composition may be administered orally, while the second composition can be administered intravenously.

The β-glucan can be administered at a dosage of, generally, from about 0.01 mg/kg/day to about 10 mg/kg/day, although the methods described herein can be practiced by administering the β-glucan at a daily dosage outside of this range. The dose of β-glucan can be from about 0.1 mg/kg/day to about 2.5 mg/kg/day. The β-glucan may be PGG.

The active agent of the second composition can be administered at a dosage of, generally, from about 100 mg/m²/week to about 800 mg/m²/week such as, for example, from about 250 mg/m²/week to about 400 mg/m²/week. Cetuximab may be administered in a first infusion at a dose of from about 200 mg/m² to about 400 mg/m², followed by a weekly infusion of from about 125 mg/m² to about 150 mg/m².

When the β-glucan composition and the second composition (e.g., an EGF-r antagonist composition) are administered together, the β-glucan composition and the second composition may be administered in a single weekly infusion. Doses of β-glucan administered per week can range from about 0.07 mg/kg/week to about 200 mg/kg/week. Dosages of EGF-r antagonist can vary. For example, cetuximab (ERBITUX, Bristol-Myers Squibb, New York, NY) is typically administered intravenously at a dose of about 400-500 mg/m² (about 10.8-13.5 mg/kg). As another example, erlotinib (TARCEVA, Genentech, South San Francisco, CA) is typically administered orally at a dose of about 100 mg/day (about 10 mg/kg/week for a 70 kg patient). The β-glucan and EGF-r antagonist may also be administered according to any suitable schedule. Suitable dosing schedules include, for example, one to three doses per week.

The method may include therapy for a proliferative disorder and includes administering PGG as the β-glucan and cetuximab as the second composition. The method may also include administering PGG, cetuximab, and irinotecan. The method may include administering PGG, cetuximab, and cisplatin. The method may include administering PGG, cetuximab, and doxorubicin.

The β-glucan composition and the second composition can be administered separately or co-formulated in a suitable co-formulated dosage form. A variety of preparations can be used to formulate pharmaceutical compositions containing β-glucan the second composition. Techniques for formulation and administration may be found in "Remington: The Science and Practice of Pharmacy, Twentieth Edition," Lippincott Williams & Wilkins, Philadelphia, PA. Suitable formulations include, for example, tablets, capsules, pills, powders, granules, dragees, gels, slurries, ointments, solutions suppositories, injections, inhalants, and aerosols. The formulations can be administered locally or systemically. Also, the formulations may be administered to provide for sustained release and/or depot of the active agent. Suitable formulations can further include one or more pharmaceutical excipients such as, for example, antioxidants, stabilizing agents, wetting agents, clarifying agents, viscosity-increasing agents, carriers, tonicity agents, buffers, preservatives, and/or encapsulation formulations.

A carrier can be a solvent or dispersion medium containing, for example, water, ethanol, polyol (for example, glycerol, propylene glycol, and liquid polyethylene glycol, and the like), vegetable oils, or mixtures or combinations thereof. The proper fluidity can be maintained by, for example, the use of a coating (e.g., lecithin), the maintenance of the required particle size in the case of dispersion, and the use of surfactants. The carrier can further include one or more antibacterial and antifungal agents such as, for example, parabens, chlorobutanol, phenol, sorbic acid, thimerosal, and mixtures or combinations thereof. In some cases, it may be desirable to include isotonic agents such as, for example, sugars or sodium chloride. Absorption of injectable compositions can be sustained by including absorption delaying agents delaying such as, for example, aluminum monostearate and gelatin in the composition.

Suitable preservatives include, for example, benzalkonium chloride, benzethonium chloride, chlorobutanol, thimerosal, and mixtures and combinations thereof. Suitable buffers include, for example, boric acid, sodium and potassium bicarbonate, sodium and potassium borates, sodium and potassium carbonate, sodium acetate, sodium biphosphate, and mixtures and combinations thereof. A buffer (or mixture or combination of buffers) can be provided in amounts sufficient to maintain the pH of the composition between about pH 6 and pH 8 such as, for example, between about pH 7 and pH 7.5. Suitable tonicity agents include, for example, dextran 40, dextran 70, dextrose, glycerin, potassium chloride, propylene glycol, sodium chloride, and mixtures or combinations thereof. A tonicity agent may be provided in an ophthalmic solution composition in an amount so the sodium chloride equivalent of the ophthalmic solution is in the range 0.9 ± 0.2%. Suitable antioxidants and stabilizers include, for example, sodium bisulfite, sodium metabisulfite, sodium thiosulfite, thiourea, and mixtures or combinations thereof. Suitable wetting and clarifying agents include, for example, polysorbate 80, polysorbate 20, poloxamer 282, tyloxapol, and mixtures or combinations thereof. Suitable viscosity-increasing agents include, for example, dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone, carboxymethylcellulose, and mixtures or combinations thereof.

The compositions can be formulated by dissolving, suspending, or emulsifying the active agent-i.e., the β-glucan, EGF-r antagonist, antibody, and/or other active agents-in an appropriate aqueous or nonaqueous solvent. Suitable nonaqueous solvents include, for example, vegetable or similar oils (e.g., sesame oil, peanut oil, etc.), synthetic aliphatic acid glycerides, esters of higher aliphatic acids, and/or propylene glycol. Suitable aqueous solutions include, for example, Hank's solution, Ringer's solution, and/or physiological saline buffer.

The compositions described herein may be formulated for subcutaneous or intramuscular injection. The solvent may be DMSO, which can result in rapid penetration and, therefore, delivery of high concentrations of the compositions to a small area.

The subject treated by the methods described herein can be a mammal such as, for example, a human, although they may also be applied to non-human mammals such as, for example, apes, monkeys, dogs, mice, etc. Thus, the methods described herein may be practiced in a veterinarian context.

The present invention is illustrated by the following examples.

### EXAMPLES

### EXAMPLE 1

A culture of *S. cerevisiae* is started and expanded stepwise through a shake flask culture into a 250 L scale production fermenter. The *S. cerevisiae* are grown in a glucose-ammonium sulfate medium enriched with vitamins (e.g., folic acid, inositol, nicotinic acid, pantothenic acid (calcium and sodium salt), pyridoxine HCl, and thymine HCl) and trace metals (e.g., from compounds such as ferric chloride, hexahydrate; zinc chloride; calcium chloride, dihydrate; molybdic acid; cupric sulfate, pentahydrate; and boric acid). An antifoaming agent such as Antifoam 204 may also be added at a concentration of about 0.02%.

The production culture is maintained under glucose limitation in a fed batch mode. During seed fermentation, samples are taken periodically to measure the optical density of the culture before inoculating the production fermenter. During production fermentation, samples are also taken periodically to measure the optical density of the culture. At the end of fermentation, samples are taken to measure the optical density, the dry weight, and the microbial purity.

If desired, fermentation may be terminated by raising the pH of the culture to at least 11.5 or by centrifuging the culture to separate the cells from the growth medium. In addition, depending on the size and form of purified β-glucan that is desired, steps to disrupt or fragment the yeast cells may be carried out. Any known chemical, enzymatic or mechanical methods, or surfactants include, for example, octylthioglucoside, Lubrol PX, Triton X-100, sodium lauryl sulfate (SDS), Nonidet P-40, Tween 20, and the like. Ionic (anionic, cationic, amphoteric) surfactants (e.g., alkyl sulfonates, benzalkonium chlorides, and the like) and nonionic surfactants (e.g., polyoxyethylene hydrogenated castor oils, polyoxyethylene sorbitol fatty acid esters, polyoxyethylene sorbitan fatty acid esters, polyoxyethylene glycerol fatty acid esters, polyethylene glycol fatty acid esters, polyoxyethylene alkyl phenyl ethers, and the like) may also be used. The concentration of surfactant will vary and depend, in part, on which surfactant is used.

Extractions are usually carried out at temperatures between about 70°C and about 90°C. Depending on the temperature, the reagents used and their concentrations, the duration of each extraction is between about 30 minutes and about 3 hours.

After each extraction, the solid phase containing the β-glucan is collected using centrifugation or continuous-flow centrifugation and resuspended for the subsequent step. The solubilized contaminants are removed in the liquid phase during the centrifugations, while the β-glucan remains in the insoluble cell wall material.

Yeast cell material may be subject to one or more extractions such as, for example, four extractions. In the first extraction, harvested yeast cells are mixed with 1.0 M NaOH and heated to 90°C for approximately 60 minutes. The second extraction is an alkaline/surfactant extraction whereby the insoluble material is resuspended in 0.1 M NaOH and about 0.5% to 0.6% Triton X-100 and heated to 90°C for approximately 120 minutes. The third extraction is an alkaline/surfactant extraction whereby the insoluble material is resuspended in 0.1 M NaOH and about 0.05% Triton X-100 and heated to 90°C for approximately 60 minutes. In the fourth extraction, the insoluble material is resuspended in about 0.5% Triton-X 100 and heated to 75°C for approximately 60 minutes.

The alkaline and/or surfactant extractions solubilize and remove some of the extraneous yeast cell materials. The alkaline solution hydrolyzes proteins, nucleic acids, mannans, and lipids. Surfactant enhances the removal of lipids and other hydrophobic impurities, which provides an additional advantage yielding an improved β-glucan product.

Fat content of the yeast *S. cerevisiae* produced by aerobic and anaerobic growth ranges from about 3% to about 8%. The fat content varies depending on growth conditions of the yeast. Table 1 provides an overview of the typical fat composition of the yeast *S. cerevisiae*. The data are from the following references:
• Blagovic, B., J. Rpcuc, M. Meraric, K. Georgia and V. Maric. 2001. Lipid composition of brewer's yeast. Food Technol. Biotechnol. 39:175-181.
• Shulze. 1995. Anaerobic physiology of Saccharomyces cerevisiae. Ph.D. Thesis, Technical University of Denmark.
• Van Der Rest, M. E., A. H. Kamming, A Nakano, Y. Anrak, B. Poolman and W. N. Koning. 1995. The plasma membrane of Saccharomyces cerevisiae: structure, function and biogenesis. Microbiol. Rev. 59:304-322.

**TABLE 1**

| Fatty acid | Shulze (1995) | Blagovic et al (2001) (anaerobic growth) | Van Der Rest et al (1995) |
|---|---|---|---|
| 10:0 Capric acid | 1.1% | | |
| 12:0 and 12:1 Lauric acid | 4.8% | 7.3% | |
| 14:0 and 14:1 Myristic acid | 8.8% | 5.1% | 7.0^{*}% |
| 16:0 Palmitic acid | 26.8% | 44.2% | 12.8% |
| 16:1 Palmitoleic acid | 16.6% | 16.9% | 32.3% |
| 18:0 Stearic acid | 6.1% | 13.9% | 8.0% |
| 18.1 Oleic acid | 25.7% | 7.3% | 28.0% |
| 18:2 and higher Linoleic acid, arachadonic acid and others | 10.1% | 5.3% | 9.4% |

| | | | |
|---|---|---|---|
| * includes lipids 10:0 to 14:1 | | | |

Yeast cell wall material typically contains 10-25% fat depending on yeast type and growth conditions. Presently, during processing of yeast cell wall material into β-glucan, some, but not all fat is removed by centrifugation and wash steps. Thus, a typical preparation might yield a fat content of 3-7%.

The manufacturing process typically involves steps to remove mannoproteins, lipids and other undesirable components of the yeast cell wall. Some key steps common to this processing are various wash steps that employ acid and alkali in separate washing steps to liberate certain cell wall components. Several of the steps use an alkali wash process where an alkali, usually sodium hydroxide, is added to the liquid cell wall suspension. One of the purposes of the alkali is to remove lipid by forming the free fatty acids of the lipid. The result is a reduction in fat content of the β-glucan.

The alkali wash steps commonly used in production of yeast β-glucan leave behind residual fatty acids and partially degraded fat triglycerides that have increased reactivity. The direct result of the alkali wash process is the release of reactive free fatty acids that quickly decompose to various oxidative products of fat decomposition.

The next step in the purification process is an acidic extraction that removes glycogen. One or more acidic extractions are accomplished by adjusting the pH of the alkaline/surfactant extracted material to between about pH 5 and pH 9 and mixing the material in about 0.05 M to about 1.0 M acetic acid at a temperature between about 70°C and 100°C for approximately 30 minutes to about 12 hours.

The insoluble material remaining after centrifugation of the alkaline/surfactant extraction is resuspended in water, and the pH of the solution is adjusted to about 7 with concentrated HCl. The material is mixed with enough glacial acetic acid to make a 0.1 M acetic acid solution, which is heated to 90°C for approximately 5 hours.

Next, the insoluble material is washed. In a typical wash step, the material is mixed in purified water at about room temperature for a minimum of about 20 minutes. The water wash is carried out two times. The purified β-glucan product is then collected. Again, collection is typically carried out by centrifugation or continuous-flow centrifugation.

At this point, a purified, particulate β-glucan product is formed. The product may be in the form of whole glucan particles or any portion thereof, depending on the starting material. In addition, larger sized particles may be broke down into smaller particles. The range of product includes β-glucan particles that have substantially retained *in vivo* morphology (whole glucan particles) down to submicron-size particles.

Particulate β-glucan can be processed further to form aqueous, soluble β-glucan.

### EXAMPLE 2

Particulate β-glucan such as, for example, particulate β-glucan as produced in EXAMPLE 1 may be further processed to form soluble β-glucan. The particulate β-glucan starting material may range in size from whole glucan particles down to submicron-sized particles.

Particulate β-glucan undergoes an acidic treatment under pressure and elevated temperature to produce soluble β-glucan. Pelleted particulate β-glucan is resuspended and mixed in a sealable reaction vessel in a buffer solution and brought to pH 3.6. Buffer reagents are added such that every liter, total volume, of the final suspension mixture contains about 0.61 g sodium acetate, 5.24 ml glacial acetic acid and 430 g pelleted particulate β-glucan. The vessel is purged with nitrogen to remove oxygen and increase the pressure within the reaction vessel.

The pressure inside the vessel is brought to 35 PSI, and the suspension is heated to about 135°C for between about 4.5 and 5.5 hours. The pressure and temperature reduces the need to use hazardous chemicals to solubilize the β-glucan and thereby improves safety and reduces costs for materials.

The exact duration of heat treatment is typically determined experimentally by sampling reactor contents and performing gel permeation chromatography (GPC) analyses. The objective is to maximize the yield of soluble material that meets specifications for high resolution-GPC (HR-GPC) profile and impurity levels, which are discussed below. Once the β-glucan is solubilized, the mixture is cooled to stop the reaction.

The crude, solubilized β-glucan may be washed and utilized in some applications at this point, however, for pharmaceutical applications further purification is performed. Any combination of one or more of the following steps may be used to purify the soluble β-glucan. The soluble β-glucan may be clarified. Suitable clarification means include, for example, centrifugation or continuous-flow centrifugation.

Next, the soluble β-glucan may be filtered using, for example, a depth filter followed by a 0.2 µm filter.

The β-glucan may be further purified by chromatography. The soluble β-glucan may be conditioned at some point during previous steps in preparation for chromatography. For example, if a chromatographic step includes hydrophobic interaction chromatography (HIC), the soluble β-glucan can be conditioned to the appropriate conductivity and pH with a solution of ammonium sulphate and sodium acetate. A suitable solution is 3.0 M ammonium sulfate, 0.1 M sodium acetate, which is used to adjust the pH to 5.5.

In one example of HIC, a column is packed with Tosah Toyopearl Butyl 650M resin (or equivalent). The column is packed and qualified according to the manufacturer's recommendations.

Prior to loading, the column equilibration flow-through is sampled for pH, conductivity and endotoxin analyses. The soluble β-glucan, conditioned in the higher concentration ammonium sulphate solution, is loaded and then washed. The soluble β-glucan will bind to the HIC column. Low molecular weight products as well as some high molecular weight products are washed through. Soluble β-glucan remaining on the column is eluted with a buffer such as 0.2 M ammonium sulfate, 0.1 M sodium acetate, pH 5.5. Multiple cycles may be necessary to ensure that the hexose load does not exceed the capacity of the resin. Fractions are collected and analyzed for the soluble β-glucan product.

Another chromatographic step that may be utilized is gel permeation chromatography (GPC). In one example of GPC, a Tosah Toyopearl HW55F resin, or equivalent is utilized and packed and qualified as recommended by the manufacturer. The column is equilibrated and eluted using citrate-buffered saline (0.14 M sodium chloride, 0.011 M sodium citrate, pH 6.3). Prior to loading, column wash samples are taken for pH, conductivity and endotoxin analyses. Again, multiple chromatography cycles may be needed to ensure that the load does not exceed the capacity of the column.

The eluate is collected in fractions, and various combinations of samples from the fractions are analyzed to determine the combination with the optimum profile. For example, sample combinations may be analyzed by HR-GPC to yield the combination having an optimized HR-GPC profile. In one optimized profile, the amount of high molecular weight (HMW) impurity, that is soluble β-glucans over 380,000 Da, is less than or equal to 10%. The amount of low molecular weight (LMW) impurity, under 25,000 Da, is less than or equal to 17%. The selected combination of fractions is subsequently pooled.

At this point, the soluble β-glucan is purified and ready for use. Further filtration may be performed in order to sterilize the product. If desired, the hexose concentration of the product can be adjusted to about 1.0 ± 0.15 mg/ml with sterile citrate-buffered saline.

The soluble β-glucan has an average molecular weight between about 120,000 Da and about 205,000 Da and a molecular weight distribution (polydispersity) of not more than 2.5 as determined by HR-GPC with multiple angle light scattering (HR-GPC/MALS) and differential refractive index detection. Powder X-ray diffraction and magic-angle spinning NMR determined that the product consists of polymeric chains associated into triple helices. The soluble β-glucan is typically uncharged and therefore has no pKₐ. It is soluble in water independent of pH, and the viscosity increases as the concentration increases.

### EXAMPLE 3

### Mice and tumor models

BALB/c nude (Nu/Nu) mice were purchased from Taconic Farms, Inc. (Hudson, NY). For the GEO colon carcinoma xenograft model, 6- to 8-week-old SCID mice were implanted subcutaneously in a mammary fatpad with 10 x 10⁶ GEO colon carcinoma cells, which contain a Gly12Ala homozygous *KRAS* mutation. When a palpable tumors reached 350 mm², animals were divided into four groups (n = 7) and treated with either saline (control), ERBITUX (Bristol-Myers Squibb, New York, NY, 0.25 mg/dose), IMPRIME PGG (Biolpolymer Engineering, Inc., Eagan, MN, 1.2 mg/dose), or ERBITUX (0.25 mg/dose) and IMPRIME PGG (1.2 mg/dose), every three days for eight doses.

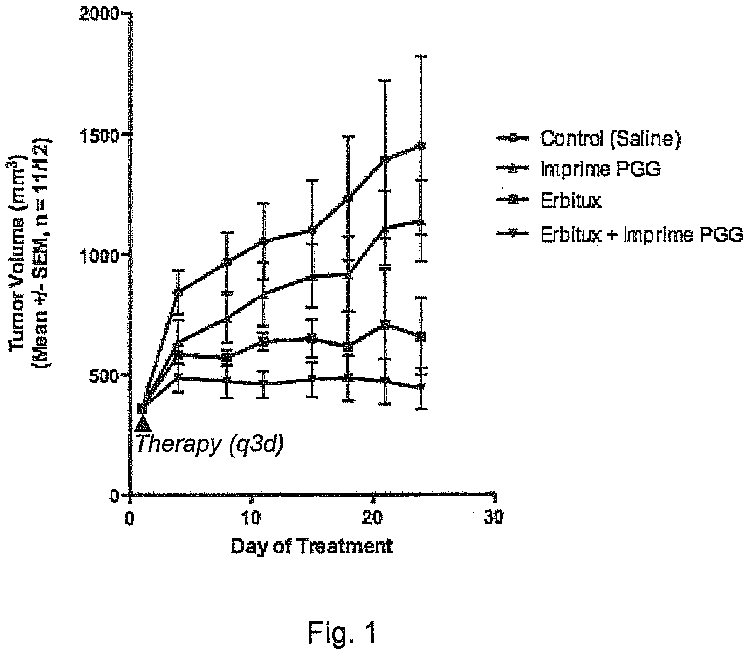

During the treatment period, tumor volumes were calculated as the average of perpendicular diameters measured using calipers. Tumor volumes were determined every three days. Mice were sacrificed when tumors reached 12 mm in diameter. Results are shown in Fig. 1.

### EXAMPLE 4

### Cell Culture

The tumor target line HCT-116 colorectal carcinoma (American Type Culture Collection, Manassas, VA, ATCC No. CCL-247) was maintained in McCoy's 5A media supplemented with 10% fetal calf serum (Hyclone Laboratories, Inc., Logan, Utah).

### Cytotoxicity Assay

Cytotoxicity was measured with the LIVE/DEAD Cell Mediated Cytotoxicity Kit (Invitrogen, Carlsbad, CA) according to manufacturer's instructions. Briefly, the HCT-116 target cells were stained with 1:80 dilution of DiOC18 in Phosphate Buffered Saline (PBS, Mediatech Inc. Manassas, VA) and incubated at 37°C for 30 minutes. The cells were then washed twice in PBS and resuspended at 2x10⁶ cells/mL in complete media containing 10% human serum.

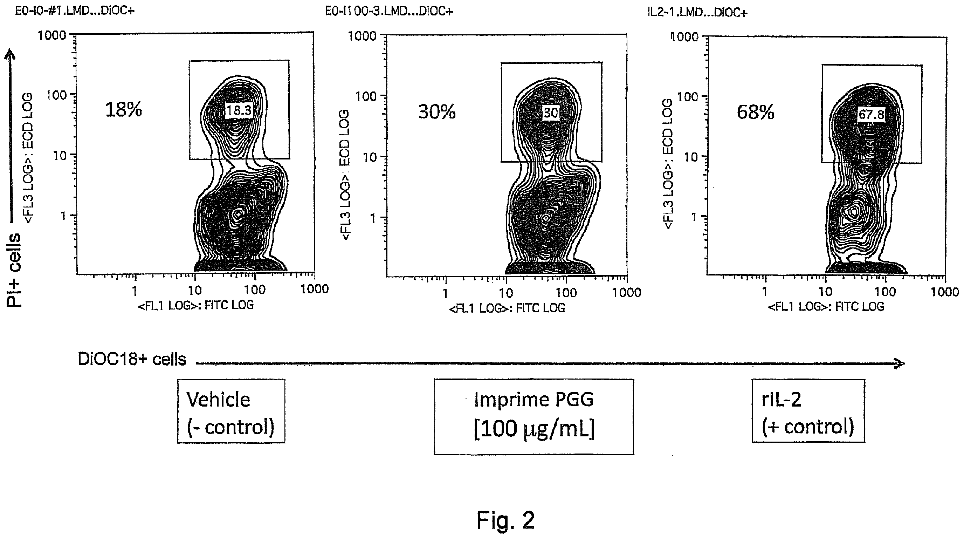

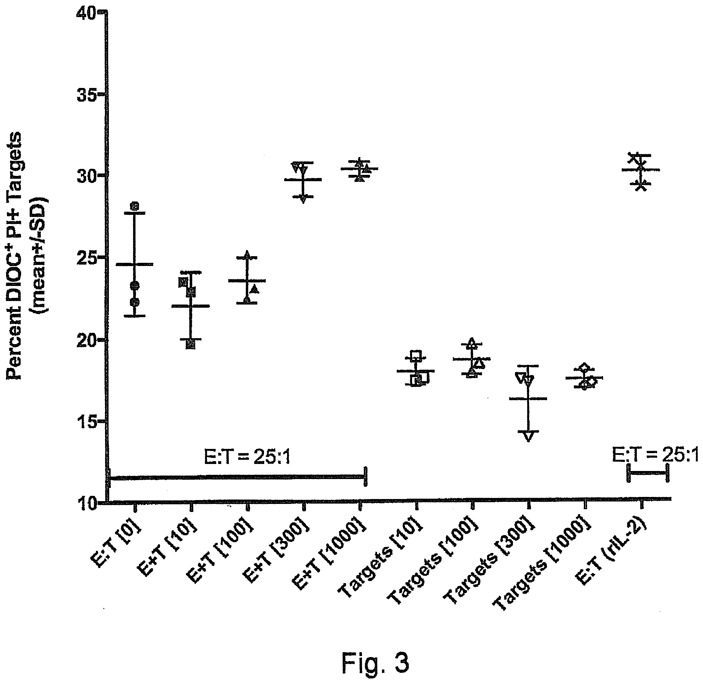

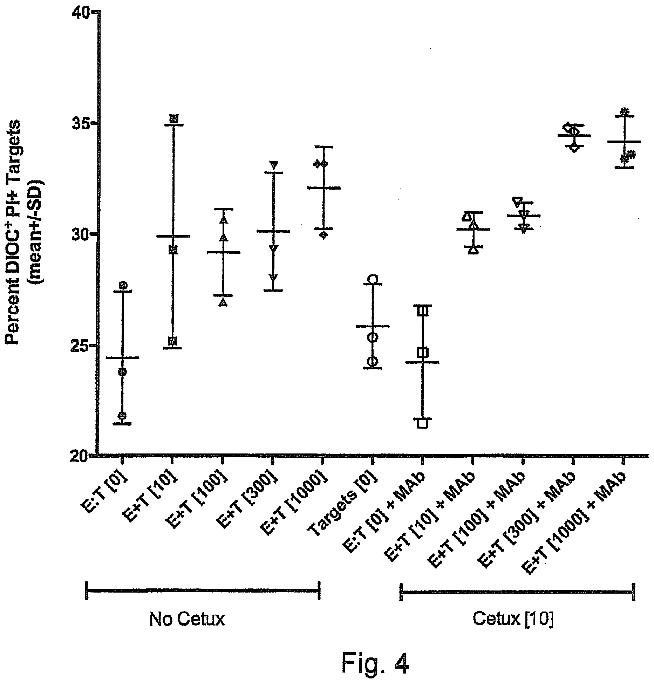

DiOC labeled target cells incubated with ERBITUX dilutions for 30 minutes at 37°C. In each well of a 96-well plate, 1.25x10⁶ freshly isolated peripheral blood mononuclear cells (PBMC) along with IMPRIME-PGG dilution or 50 ng/ml IL-2 (R&D Systems, Minneapolis, MN) in of total volume of 100 µl media incubated at 37°C for 30 minutes. 5x10⁴ targets added to wells in 25 µl of media and assay incubated overnight at 37°C. Following incubation, target cell viability measured by staining with 125 µl of propidium iodide (PI) viability reagent diluted 1:5000 from stock solution provided in the assay kit. Cells incubated for 30 minutes at 37°C before analysis on LSR II flow cytometer (BD Biosciences, San Jose, CA) to determine percentage of DiOC stained targets that intercalate the PI stain, indicating cell death. Results are shown in FIGS. 2-4.

## Claims

1. A composition comprising a ß-glucan and
a second composition that includes at least one of:
an epidermal growth factor receptor (EGF-r) antagonist; and
an antibody that binds to EGF-r;
for the use in treating a subject having a tumor comprising KRAS-mutated cells; wherein each composition is used in an amount that, in combination with the other composition, is effective to ameliorate at least one symptom or clinical sign of the tumor;
with the proviso that the KRAS-mutated cells are not SKOV 3 cells or NCI H23 cells.

2. The compositions for use according to claim 1, wherein the second composition comprises an antibody that binds to EGF-r.

3. The compositions for use according to claim 1, wherein the second composition comprises an EGF-r antagonist.

4. A composition comprising a ß-glucan for use in improving treatment of a tumor in a subject, wherein said subject is treated with an EGF-r antagonist, and wherein said subject comprises neoplastic cells comprising a KRAS mutation; with the proviso that the condition does not comprise a tumor that comprises SKOV-3 cells or NCI-H23 cells.

5. The compositions for use according to claim 1 or 2, wherein the composition that comprises the ß-glucan further comprises the second composition.

6. The compositions for use according to claim 2, wherein the antibody composition comprises cetuximab, panitumumab, rituximab, bevacizumab, trastuzumab, or alemtuzumab.

7. The composition for use according to claim 4, wherein the composition comprising a ß-glucan further comprises the EGF-r antagonist.

8. The composition for use according to claim 4, wherein the EGF-r antagonist comprises antibody that specifically binds EGF-r.

9. The compositions for use according to claim 3, wherein the first composition and the second composition are combined prior to being administered to the subject.

10. The composition(s) for use according to any one of claims 2 to 4, wherein the condition comprises colon carcinoma, colorectal carcinoma, lung adenocarcinoma, lung squamous cell carcinoma, lung large cell carcinoma, intraductal papillary mucinous tumor, or mucinous cystic tumor.

11. The composition(s) for use according to any one of claims 2 to 4, wherein the KRAS-mutated cells comprise at least one of: GEO cells and HCT-115 cells.

12. The compositions for use according to claim 3, wherein the EGF-r antagonist comprises antibody.

13. The composition(s) for use according to any one of claims 8 or 12, wherein the antibody comprises a monoclonal antibody.

14. The composition(s) for use according to claim 13, wherein the monoclonal antibody comprises a humanized monoclonal antibody.

15. The composition(s) for se according to any one of claims 8 or 12, wherein the antibody comprises a recombinant antibody.

16. The composition(s) for use according to any one of claims 8 or 12, wherein the antibody composition comprises cetuximab or panitumumab.

17. The composition(s) for use according to any one of claims 2, 4 or 12, wherein administering the first composition to the subject enhances killing of the KRAS-mutated tumor cells by PBMCs.

18. The compositions for use according to claim 2 for use in treating a subject having a tumor comprising KRAS-mutated cells, wherein the subject has been identified as having a tumor comprising KRAS-mutated cells prior to the treatment.

19. The compositions for use according to claim 3 for use in treating a subject having a tumor comprising KRAS-mutated cells, wherein the subject has been identified as having a tumor comprising KRAS-mutated cells prior to the treatment.

## Patentansprüche

1. Zusammensetzung umfassend ein β-Glucan und
und eine zweite Zusammensetzung, die mindestens einen Stoff beinhaltet von:
einem epidermalen Wachstumsfaktor-Rezeptor(EGF-r)-Antagonisten; und
einem Antikörper, der an EGF-r bindet;
zur Verwendung in der Behandlung eines Individuums, das einen Tumor hat, der KRAS-mutierte Zellen umfasst, wobei jede Zusammensetzung in einer Menge verwendet wird, die in Kombination mit der anderen Zusammensetzung effektiv mindestens ein Symptom oder klinisches Anzeichen des Tumors verbessert;
unter der Bedingung, dass die KRAS-mutierten Zellen keine SKOV 3-Zellen oder NCI H23 Zellen sind.

2. Zusammensetzungen zur Verwendung nach Anspruch 1, wobei die zweite Zusammensetzung einen Antikörper umfasst, der an EGF-r bindet.

3. Zusammensetzungen zur Verwendung nach Anspruch 1, wobei die zweite Zusammensetzung einen EGF-r-Antagonisten umfasst.

4. Zusammensetzung umfassend ein β-Glucan zur Verwendung in der Verbesserung der Behandlung eines Tumors in einem Individuum, wobei das Individuum mit einem EGF-r-Antagonisten behandelt wird und wobei das Individuum neoplastische Zellen aufweist, die eine KRAS-Mutation enthalten, unter der Bedingung, dass der Zustand keinen Tumor umfasst, der SKOV 3-Zellen oder NCI-H23 Zellen umfasst.

5. Zusammensetzungen zur Verwendung nach Anspruch 1 oder 2, wobei die Zusammensetzung, die das β-Glucan umfasst, weiterhin die zweite Zusammensetzung umfasst.

6. Zusammensetzungen zur Verwendung nach Anspruch 2, wobei die Antiköper-Zusammensetzung Cetuximab, Panitumumab, Rituximab, Bevacizumab, Trastuzumab oder Alemtuzumab umfasst.

7. Zusammensetzung zur Verwendung nach Anspruch 4, wobei die die Zusammensetzung, die das β-Glucan umfasst, weiterhin den EGF-r-Antagonisten umfasst.

8. Zusammensetzung zur Verwendung nach Anspruch 4, wobei der EGF-r-Antagonist einen Antikörper umfasst, der EGF-r spezifisch bindet.

9. Zusammensetzungen zur Verwendung nach Anspruch 3, wobei die erste Zusammensetzung und die zweite Zusammensetzung vor der Verabreichung an das Individuum kombiniert werden.

10. Zusammensetzung(en) zur Verwendung nach einem der Ansprüche 2 bis 4, wobei der Zustand Colonkarzinom, colorektales Karzinom, Lungenadenokarzinom, Lungen-Plattenepithelkarzinom, großzelliges Lungenkarzinom, intraduktalen papillären Gallerttumor oder cystischen Gallerttumor umfasst.

11. Zusammensetzung(en) zur Verwendung nach einem der Ansprüche 2 bis 4, wobei die KRAS-mutierten Zellen mindestens eine von folgenden Zellarten umfassen: GEO-Zellen und HTC-115-Zellen.

12. Zusammensetzungen zur Verwendung nach Anspruch 3, wobei der EGF-r-Antagonist einen Antikörper umfasst.

13. Zusammensetzung(en) zur Verwendung nach einem der Ansprüche 8 oder 12, wobei der Antikörper einen monoklonalen Antikörper umfasst.

14. Zusammensetzung(en) zur Verwendung Anspruch 13, wobei der monoklonale Antikörper einen humanisierten monoklonalen Antikörper umfasst.

15. Zusammensetzung(en) zur Verwendung nach einem der Ansprüche 8 oder 12, wobei der Antikörper einen rekombinanten Antikörper umfasst.

16. Zusammensetzung(en) zur Verwendung nach einem der Ansprüche 8 oder 12, wobei die Antikörperzusammensetzung Cetuximab oder Panitumumab umfasst.

17. Zusammensetzung(en) zur Verwendung nach einem der Ansprüche 2, 4 oder 12, wobei die Verabreichung der ersten Zusammensetzung an das Individuum die Abtötung von KRAS-mutierten Tumorzellen durch PBMCs verstärkt.

18. Zusammensetzungen zur Verwendung nach Anspruch 2 zur Verwendung in der Behandlung eines Individuums, das einen Tumor umfassend KRAS-mutierte Zellen hat, wobei das Individuum vor der Behandlung als einen Tumor tragend identifiziert wurde, der KRAS-mutierte Zellen umfasst.

19. Zusammensetzungen zur Verwendung nach Anspruch 3 zur Verwendung in der Behandlung eines Individuums, das einen Tumor umfassend KRAS-mutierte Zellen hat, wobei das Individuum vor der Behandlung als einen Tumor tragend identifiziert wurde, der KRAS-mutierte Zellen umfasst.

## Revendications

1. Composition comprenant un β-glucane et
une deuxième composition qui contient au moins l'un parmi:
un antagoniste de récepteur de facteur de croissance épidermique (EGF-r); et
un anticorps qui se lie à EGF-r;
pour l'utilisation dans le traitement d'un sujet ayant une tumeur comprenant des cellules avec une mutation KRAS; dans laquelle chaque composition est utilisée en une quantité qui, en combinaison avec l'autre composition, est efficace pour améliorer au moins un symptôme ou signe clinique de la tumeur;
sous réserve que les cellules avec une mutation KRAS ne soient pas des cellules SKOV 3 ou des cellules NCI H23.

2. Compositions pour une utilisation selon la revendication 1, dans lesquelles la deuxième composition comprend un anticorps qui se lie à EGF-r.

3. Compositions pour une utilisation selon la revendication 1, dans lesquelles la deuxième composition comprend un antagoniste d'EGF-r.

4. Composition comprenant un β-glucane pour une utilisation dans l'amélioration du traitement d'une tumeur chez un sujet, dans laquelle ledit sujet est traité avec un antagoniste d'EGF-r, et dans laquelle ledit sujet comprend des cellules néoplasiques comprenant une mutation KRAS; sous réserve que l'état ne comprenne pas une tumeur qui comprend des cellules SKOV-3 ou des cellules NCI-H23.

5. Compositions pour une utilisation selon la revendication 1 ou 2, dans lesquelles la composition qui comprend le β-glucane comprend en outre la deuxième composition.

6. Compositions pour une utilisation selon la revendication 2, dans lesquelles la composition d'anticorps comprend du cétuximab, du panitumumab, du rituximab, du bévacizumab, du trastuzumab, ou de l'alemtuzumab.

7. Composition pour une utilisation selon la revendication 4, dans laquelle la composition comprenant un β-glucane comprend en outre l'antagoniste d'EGF-r.

8. Composition pour une utilisation selon la revendication 4, dans laquelle l'antagoniste d'EGF-r comprend un anticorps qui se lie spécifiquement à EGF-r.

9. Compositions pour une utilisation selon la revendication 3, dans lesquelles la première composition et la deuxième composition sont combinées avant d'être administrées au sujet.

10. Composition(s) pour une utilisation selon l'une quelconque des revendications 2 à 4, dans lesquelles l'état comprend un carcinome du côlon, un carcinome colorectal, un adénocarcinome pulmonaire, un carcinome pulmonaire à cellules squameuses, un carcinome pulmonaire à grosses cellules, une tumeur mucoïde papillaire intraductale, ou une tumeur kystique mucoïde.

11. Composition(s) pour une utilisation selon l'une quelconque des revendications 2 à 4, dans lesquelles les cellules avec une mutation KRAS comprennent au moins l'une parmi: les cellules GEO et les cellules HCT-115.

12. Compositions pour une utilisation selon la revendication 3, dans lesquelles l'antagoniste d'EGF-r comprend un anticorps.

13. Composition(s) pour une utilisation selon l'une quelconque des revendications 8 ou 12, dans lesquelles l'anticorps comprend un anticorps monoclonal.

14. Composition(s) pour une utilisation selon la revendication 13, dans lesquelles l'anticorps monoclonal comprend un anticorps monoclonal humanisé.

15. Composition(s) pour une utilisation selon l'une quelconque des revendications 8 ou 12, dans lesquelles l'anticorps comprend un anticorps recombinant.

16. Composition(s) pour une utilisation selon l'une quelconque des revendications 8 ou 12, dans lesquelles la composition d'anticorps comprend du cétuximab ou du panitumumab.

17. Composition(s) pour une utilisation selon l'une quelconque des revendications 2, 4 ou 12, dans lesquelles l'administration de la première composition au sujet amplifie la destruction par les PBMC des cellules tumorales avec une mutation KRAS.

18. Compositions pour une utilisation selon la revendication 2, pour une utilisation dans le traitement d'un sujet ayant une tumeur comprenant des cellules avec une mutation KRAS, dans lesquelles le sujet a été identifié comme ayant une tumeur comprenant des cellules avec une mutation KRAS avant le traitement.

19. Compositions pour une utilisation selon la revendication 3, pour une utilisation dans le traitement d'un sujet ayant une tumeur comprenant des cellules avec une mutation KRAS, dans lesquelles le sujet a été identifié comme ayant une tumeur comprenant des cellules avec une mutation KRAS avant le traitement.
